(19) Europäisches Patentamt
European Patent Office
Office européen des brevets

(11) Veröffentlichungsnummer: **0 120 396**
**B1**

(12) **EUROPÄISCHE PATENTSCHRIFT**

(45) Veröffentlichungstag der Patentschrift: **30.05.90**

(21) Anmeldenummer: **84102708.9**

(22) Anmeldetag: **13.03.84**

(51) Int. Cl.$^5$: **C 12 Q 1/00**, C 12 Q 1/34, G 01 N 33/50

# (54) Teststreifen zum Nachweis von Leukozyten im Harn.

(30) Priorität: **17.03.83 DE 3309544**

(43) Veröffentlichungstag der Anmeldung:
**03.10.84 Patentblatt 84/40**

(45) Bekanntmachung des Hinweises auf die Patenterteilung:
**30.05.90 Patentblatt 90/22**

(84) Benannte Vertragsstaaten:
**BE DE FR GB**

(56) Entgegenhaltungen:
**EP-A-0 039 880**
**DE-A-2 905 531**
**DE-A-3 227 893**

(73) Patentinhaber: **MACHEREY-NAGEL & Co. Chemikalienhandel**
**Werkstrasse 6-8**
**D-5160 Düren (DE)**

(72) Erfinder: **Flegler, Karl-Heinz, Dr.**
**Stockheimer Weg 11**
**D-5166 Kreuzau (DE)**

(74) Vertreter: **Klöpsch, Gerald, Dr.-Ing.**
**An Gross St. Martin 6**
**D-5000 Köln 1 (DE)**

**Die Akte enthält technische Angaben, die nach dem Eingang der Anmeldung eingereicht wurden und die nicht in dieser Patentschrift enthalten sind.**

EP 0 120 396 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Verwendung von Benzimidazolderivaten zum Nachweis von Leukozyten in Körperflüssigkeiten und Ausscheidungsprodukten, insbesondere als Diagnostikum in Form eines Testpapiers.

Zur frühzeitigen Erkennung der akuten und chronischen Pyelonephritis, vieler entzündlicher Erkrankungen der ableitenden Harnwege wie Urethritis, Cystitis sowie Infektionen durch Trichomonaden, Gonokokken, Mycoplasmen, Viren und nahezu allen Arten von Harntransportstörungen wird der Leukozyturie große Bedeutung beigemessen. Als besonders geeignet für einen Screening-Test haben sich Testpapiere erwiesen, die zur Reaktion die esterolytische bzw. proteolytische Aktivität der in Leukozyten vorhandenen Esterasen bzw. Proteasen ausnutzen.

Mit einem solchen Test werden neben intakten Leukozyten auch lysierte Zellen nachgewiesen, die durch mikroskopisches Auszählen der Leukozyten im nicht zentrifugierten Harn bzw. Harnsediment nicht erfaßt würden und insbesondere bei längeren Harnstandzeiten durch eine Lyse der leukozyten falsch negative Befunde zur Folge hätten.

Als Chromogene für einen solchen Test finden farblose oder schwach gefärbte Ester Verwendung, bei denen durch die enzymatische Spaltung eine Alkohol- bzw. Phenolkomponente freigesetzt wird, die selbst gefärbt ist oder in einer Folgereaktion farbige Derivate bildet. Solche Esterasesubstrate sind aus der Histochemie schon lange bekannt und werden z.B. beschrieben bei:

M. M. Nachlas, A. M. Seligman, J. Nat. Cancer Inst. *9*, 415 (1949)
R. J. Barnett, A. M. Seligman, Science *114*, 579 (1951)
T. K. Shnitka, A. M. Seligman, J. Histochem. *9*, 504 (1961)
F. Schmalzl, H. Braunsteiner, Klin. Wschr. *46*, 642 (1968)
L. T. Yam, C. Y. Li, W. H. Crosby, Am. J. Clin. Pathol. *55*, 283 (1971)
M. J. Castillo, K. Nakagima, M. Zimmerman, J. C. Powers, Anal. Biochem. *99*, 53 (1979).

Weitere Bestandteile eines Testpapiers für Leukozyten sind neben dem Chromogen ein Puffersystem, ggfs. ein oberflächenaktives Mittel, ein Oxidationsmittel sowie Hilfsmittel mit verschiedenen Funktionen.

In den DE-OS 28 26 965, 28 36 644 und 38 54 987 werden Teststreifen beschrieben, die zum Nachweis einer erhöhten Leukozyturie geeignet sein sollen. Die danach hergestellten Teststreifen waren jedoch entweder zu unempfindlich oder erforderten sehr lange Reaktionszeiten (15 min und mehr).

Es ist auch bereits bekannt, die Reaktionszeit durch Kupplung der durch Esteraseabspaltung gebildeten Alkohol- bzw. Phenolkomponente mit einem Diazoniumsalz gemäß DE-OS 30 17 721 zu verkürzen. Auch kann die Reaktionszeit den Einsatz zusätzlicher Reaktionsverstärker gemäß DE—OS 29 05 531 verkürzt werden; als Reaktionsverstärker werden in der DE—OS 29 05 531 Pyridinderivate eingesetzt.

Es wurde nun überraschenderweise gefunden, daß als Aktivator außer diesen bekannten Verbindungen Benzimidazolderivate besonders gut geeignet sind. Bestimmte Benzimidazolderivate führen neben einer Erhöhung der Stabilität auch zu einer entschiedenden Verkürzung der Reaktionszeit.

Gegenstand der Erfindung ist daher die Verwendung einer Verbindung der chemischen Formel

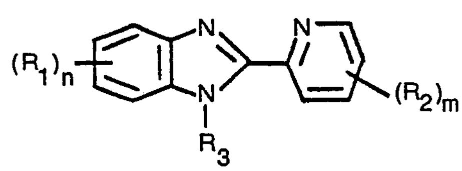

worin

$R_1$ und $R_2$ gleich oder verschieden sind und Wasserstoff, Halogen, eine niedere Alkyl- oder Alkoxygruppe mit 1 bis 4 C-Atomen, eine ($C_1$—$C_4$)-Dialkylamino-, eine Acetylaminogruppe, eine Nitrogruppe oder einen gegebenenfalls substituierten oder annellierten Aromaten bedeuten,

n eine ganze Zahl von 1 bis 4, bevorzugt 1 bis 2, und

m ebenfalls eine ganze Zahl von 1 bis 4, bevorzugt 1 bis 2 ist, und

$R_3$ für Wasserstoff, eine niedere Alkylgruppe mit 1 bis 4, bevorzugt den Acetylrest, oder einen Cyanoalkylrest, bevorzugt den Cyanoethylrest, steht oder eines Salzes dieser Verbindung in einem Mittel, insbesondere zum Nachweis von Leukozyten und anderen esterolytisch und/oder proteolytisch wirksamen Substanzen in Körperflüssigkeiten und Ausscheidungen, insbesondere auf einem saugfähigen Träger zusammen mit einem enzymspezifischen Substrat und/oder Chromogen sowie weiteren Hilfsmitteln wie einem Puffersystem, ggfs. Verdickungsmitteln, oberflächenaktiven Mitteln, Oxidationsmitteln und/oder Stabilisatoren.

Gegenstand der Erfindung ist ferner ein Diagnostikum, insbeondere Schnelldiagnostikum in Form eines Teststreifens, bestehend aus einem Träger, einem enzymspezifischen Substrat und/oder Chromogen, einem Aktivator sowie weiteren Hilfsmitteln wie einem Puffersystem, ggfs. Verdickungsmitteln, oberflächenaktiven Mitteln, Oxidationsmitteln und/oder weiteren Stabilisatoren, das dadurch gekennzeichnet ist, daß der Aktivator eine Verbindung nach Anspruch 1 ist.

Die 2-(2-Pyridyl)-benzimidazole der allgemeinen Formel sind bekannte Verbindungen. Ihre Herstellung wird z.B. beschrieben in den folgenden Literaturstellen:

V. J. Cohen, J. Heterocycl. Chem. *16*, 13 (1979)

A. Bistrzycki, A. Lecco, Helv. chim. Acta *4*, 425 (1921)

D. Jerchel, H. Fischer, M. Kracht, Liebigs Ann. *575* 162 (1952)

J. Hall, D. R. Kamm, J. Org. Chem. *30* (6), 2092 (1965)

F. H. Case, J. Heterocycl. Chem. *4*, 157 (1967)

M. Ichikawa et al., Chem. Pharm. Bull. *25* (2), 358 (1977) CA 87, 23155 q

M. Ichikawa, Org. Prep. Proced. Int. *10* (5), 205 (1978) CA *89*, 179 920 n

R. D. Haugewitz et al., J. Med. Chem. *22*, 1113 (1979) CA *91*, 117 155 z

M. Ichikawa, Chem. Pharm. Bull. *27* (5), 1255 (1979) CA *91*, 211 323 z

Als Chromogene kommen Enzymsubstrate oder andere zum Nachweis esterolytisch bzw. proteolytisch wirksamer Enzyme geeignete Substrate infrage, z.B. Sulfonphthaleinsäureester gemäß DE—OS 28 26 965, Azofarbstoffester gemäß DE—OS 28 36 644 sowie Indoxyl- und/oder Thioindoxylaminosäureester und/oder -peptidester gemäß DE—OS 28 54 987.

Die Chromogene werden üblicherweise in Konzentrationen von $10^{-4}$ bis $10^{-1}$ Mol/l Imprägnierlösung eingesetzt.

Als Puffer kommen alle Systeme infrage, die nach dem Eintauchen in die zu untersuchende Flüssigkeit einen pH-Wert von 6 bis 10, vorzugsweise 7 bis 9, ergeben. Beispiele für geeignete bekannte Puffersysteme sind Phosphat-, Barbiturat-, Borat-, Tris-(Hydroxymethyl)-Aminomethan(Tris)-, und Aminosäurepuffer.

Vorteilhaft werden erfindungsgemäß zusätzlich oberflächenaktive Mittel mit verwendet. Geeignet hierfür sind alle bei der Herstellung von Teststreifen üblicherweise eingesetzten Tenside oder Netzmittel, z.B. kationaktive Netzmittel wie quaternäre Ammoniumsalz, anionaktive Netzmittel wie Natriumlaurylsulfat, Dioctylnatriumsulfosuccinat oder nicht-ionogene Netzmittel wie z.B. Brij 35, Triton X 100® usw.

Als Oxidationsmittel können erfindungsgemäß u.a. Kaliumbromat, Kaliumjodat, Kaliumchromat, Kaliumhexacyanoferrat-(III), Phenazinmethosulfat und/oder Tetrazoliumsalze eingesetzt werden.

Als weitere Hilfsmittel können Verdickungsmittel wie Gelatine, Gummi arabicum oder auch Polyvinylpyrrolidon eingesetzt werden. Weiter seien als Hilfsstoffe noch Stabilisatoren wie z.B. Phosphor- bzw. Phosphonsäureamide und als Komplexbildner Metallsalze z.B. der Elemente Eisen, Kupfer, Zink usw. genannt.

Das Diagnostikum wird hergestellt, indem überlicherweise ein saugfähiger Träger wie ein Filtrierpapier, ein Cellulosevlies oder ein Vlies aus Synthesefaser mit einer einzigen Lösung der Komponenten oder mit mindestens zwei Lösungen getränkt und anschließend getrocknet wird, wobei bei mehrstufiger Imprägnierung jeweils vor der nachfolgenden Imprägnierung getrocknet wird.

Die Erfindung wird durch die folgend Beispiele veranschaulicht:

Beispiel 1

Es wird eine Lösung aus folgenden Bestandteilen hergestellt:

Lösung 1

6 g Natriumdihydrogenphosphat-2-hydrat

60 ml dest. Wasser

(mit 32% NaOH auf pH 8 einstellen und mit dest Wasser auf 100 ml auffüllen).

Filtrierpapier MN 215 wird mit vorstehender Lösung imprägniert und bei ca. 70°C getrocknet. Das so erhaltene Papier wird anschliessend mit folgender Lösung nachimprägniert:

Lösung 2

1 g Phosphorsäuretrimorpholid

0,25 g 2,4-Dimethoxy-benzoldiazoniumfluoroborat

0,04 g 3-[N-(Toluolsulfonyl)-L-alanyloxy]-1-methoxy naphathalin

0,25 g 2-(2-Pyridyl)-benzimidazol

100 ml Aceton

Das so erhaltene Testpapier wird zur Herstellung des Prüfmittels in 5×6 mm große Testzonen mit Hilfe geeigneter Kleber auf einen Polyesterstreifen befestigt. Die Testzone verfärbt sich beim Eintauchen in leukozytenhaltige Urine rötlich bis rot.

Beispiel 2

Filtrierpapier MN 215 wird gemäß Beispiel 1 mit nachfolgenden Lösungen nacheinander imprägniert und getrocknet.

Lösung 1

33 mg Kaliumhexacyanoferrat-(III)

40 ml Methkmol

60 ml Phosphatpuffer (s. Beispiel 1)

Lösung 2

1 g Phosphorsäuretrimorpholid
0,25 g 2-(2-Pyridyl)-benzimidazol
0,04 g 3-[N-(Toluol-4-sulfonyl)-L-alanyloxy]-indol
100 ml Aceton

Die so erhaltenen Testpapiere färben sich beim Eintauchen in leukozytenhaltige Urine blau.

Beispiel 3

Filtrierpaper MN 215 wird gemäß Beispiel 1 mit nachfolgenden Lösungen imprägniert und getrocknet:
Phosphatpuffer siehe Beispiel 1

Lösung 2

1 g Phosphorsäuretrimorpholid
0,25 g 2,4-Dimethoxy-benzoldiazoniumfluorborat
0,04 g 3-[N-(Toluolsulfonyl)-L-alanyloxy]-methoxy-naphthalin
0,25 g 5-(6)-Methyl-2-(2-pyridyl)-benzimidazol
100 ml Aceton

Das so erhaltene Testpapier färbt sich beim Eintauchen in leukozytenhaltige Urine rötlich bis rot.

In analoger Weise lassen sich anstelle des 5-(6)-Methyl-2-(2-pyridyl)-benzimidazols auch die folgenden funktionellen Derivate einsetzen:

4,5-Dimethyl-2-(2-pyridyl)-benzimidazol
5-(6)-Nitro-2-(2-pyridyl)-benzimidazol

Beispiel 4

Filtrierpapier MN 215 wird gemäß Beispiel 1 mit nachfolgenden Lösungen nacheinander imprägniert und getrocknet:

Lösung 1

0,25 g 2-(2-Pyridyl)-benzimidazol
40 ml Methanol
60 ml Phosphatpuffer pH 8 (s. Beispiel 1).

Lösung 2

1 g Phosphorsäuretrimorpholid
1 g Oleylalkohol
0,25 g 2,4-Dimethoxy-benzoldiazoniumfluoroborat
0,04 g 3-[N-(Toluolsulfonyl)-L-alanyloxy]-1-methoxy-naphthalin
100 ml Aceton

Das so erhaltene Testpapier färbt sich beim Eintauchen in leukozytenhaltige Urine rötlich bis rot.

Beispiel 5

Filtrierpapier MN 215 wird gemäß Beispiel 1 mit nachfolgenden Lösungen nacheinander imprägniert und getrocknet:

Lösung 1

Phosphatpuffer gemäß Beispiel 1

Lösung 2

1 g Phosphorsäuretrimorpholid
0,25 g 2,4-Dimethoxy-benzoldiazoniumfluoroborat
0,04 g 3-[N-(Toluolsulfonyl)-L-alanyloxy]-1-methoxy-naphthalin
0,25 g 2-(4-Methylpyridyl-2)-benzimidazol
100 ml Aceton

Das so erhaltene Testpapier färbt sich bei Eintauchen in leukozytenhaltige Urine rötlich bis rot.

In analoger Weise lassen sich anstelle des 2-(4-Methylpyridyl-2)-benzimidazols auch die folgenden Derivate einsetzen:

2-(4-Phenylpyridyl-2)-benzimidazol
1-Methyl-2-(2-pyridyl)-benzimidazol
5-Phenyl-2-(2-pyridyl)benzimidazol

Beispiel 6

Fitrierpapier MN 215 wird gemäß Beispiel 1 mit nachfolgenden Lösungen imprägniert und getrocknet:

Lösung 1

Phosphatpuffer gemäß Beispiel 1

Lösung 2

1 g Phosphorsäuretrimorpholid
0,25 g 2,4-Dimethoxy-benzoldiazoniumfluoroborat
0,04 g 3-[N-(Toluol-4-sulfonyl)-l-alanyloxy]-indol
0,25 g 5-Methoxy-2-(2-pyridyl)benzimidazol
100 ml Aceton

Das so erhaltene Testpapier färbt sich beim Eintauchen in leukozytenhaltigen Urine rötlichbraun.
In analoger Weise lässt sich auch
5-Chlor-2-(pyridyl)benzimidazol
als Aktivator einsetzen.

**Patentansprüche**

1. Verwendung einer Verbindung der chemischen Formel

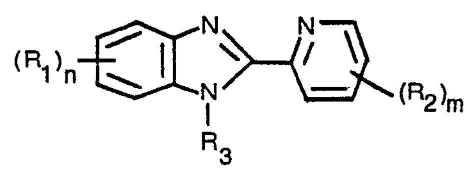

worin

$R_1$ und $R_2$ gleich oder verschieden sind und Wasserstoff, Halogen, eine niedere Alkyl- oder Alkoxygruppe mit 1 bis 4 C-Atomen, eine ($C_1$—$C_4$)-Dialkylamino- eine Acetylaminogruppe, eine Nitrogruppe oder einen gegebenenfalls substituierten oder annelierten Aromaten bedeuten,

n eine ganze Zahl von 1 bis 4, bevorzugt 1 bis 2, und

m ebenfalls eine ganze Zahl von 1 bis 4, bevorzugt 1 bis 2 ist, und

$R_3$ für Wasserstoff, eine niedere Alkylgruppe mit 1 bis 4, bevorzugt den Acetylrest, oder einen Cyanoalkylrest, bevorzugt den Cyanoethylrest, steht oder eines Salzes dieser Verbindung zum Nachweis von Leukozyten und anderen esterolytisch und/oder proteolytisch wirksamen Substanzen in Körperflüssigkeiten und Ausscheidungen, insbesondere auf einem saugfähigen Träger zusammen mit einem enzymspezifischen Substrat und/oder Chromogen sowie weiteren Hilfsmitteln wie einem Puffersystem, ggfs. Verdickungsmitteln, oberflächenaktiven Mitteln, Oxidationsmitteln und/oder Stabilisatoren.

2. Diagnostikum, insbesondere Schnelldiagnostikum in Form eines Teststreifens, bestehend aus einem Träger, einem enzymspezifischen Substrat und/oder Chromogen, zum Nachweis esterolytisch bzw. Proteolytisch wirksammer Enzyme, einem Aktivator sowie weiteren Hilfsmitteln wie einem Puffersystem, ggfs. Verdickungsmitteln, oberflächenaktiven Mitteln, Oxidationsmitteln und/oder weiteren Stabilisatoren, dadurch gekennzeichnet, daß der Aktivator eine Verbindung nach Anspruch 1 ist.

**Revendications**

1. Utilisation d'un composé de la formule chimique suivante:

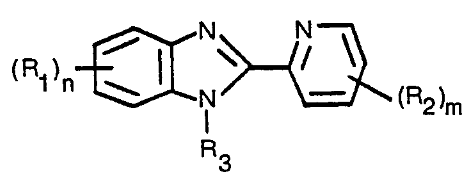

dans laquelle

$R_1$ et $R_2$ sont identiques ou différents et représentent chacun un atome d'hydrogène, un atome d'halogène, un radical alkyle ou alcoxy inférieur comportant de 1 à 4 atomes de carbone, un radical dialkyl ($C_1$—$C_4$)amino, acétylamino, nitro, ou aromatique éventuellement substitué ou annelé,

n représente un nombre entier dont la valeur varie de 1 à 4 et est, de préférence, égal à 1 ou 2 et

m représente également un nombre entier dont la valeur varie de 1 à 4 et est, de préférence, égal à 1 ou 2 et

$R_3$ représente un atome d'hydrogène, un radical alkyle comportant de 1 à 4 atomes de carbone, de préférence le radical acétyle, ou un radical cyanoalkyl, de préférence le radical cyanoéthyle, ou un sel de ce composé, pour la détection de leucocytes et d'autres substances à activité estérolytiques et/ou protéolytiques dans les liquides corporels et des excrétions plus particulièrement sur un support absorbant, conjointement avec un chromogène et/ou un substrat enzymo-spécifique, comme aussi

d'autres adjuvants, tels qu'un système tampon, éventuellement des agents épaississants, des agents tensioactifs, des agents oxydants et/ou des stabilisants.

2. Agent de diagnostic, plus particulièrement de diagnostic rapide sous la forme d'une bandelette d'essai, se composant d'un support, d'un chromogène et/ou d'un substrat enzymo spécifique, pour la détection d'enzymes à activité estérolytique ou protéolytique, d'un activateur comme aussi d'autres adjuvants, tels qu'un système tampon, éventuellement des agents épaississants, des agents tensio actifs, des agents oxydants et/ou d'autres stabilisants caractérisés en ce que l'activateur est un composé suivant la revendication 1.

## Claims

1. Use of a compound of the chemical formula

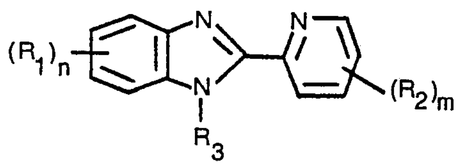

wherein

$R_1$ and $R_2$ are the same or different and represent hydrogen, halogen, a low alkyl or alkoxy group having 1 to 4 carbon atoms, a $(C_1—C_4)$-dialkylamino group, an acetylamino group, a nitro group, or an optionally substituted or anelated aromatic,

n is an integer number from 1 to 4, preferably 1 to 2, and

m is an integer number from 1 to 4, preferably 1 to 2, too, and

$R_3$ represents hydrogen, a low alkyl group having 1 to 4, preferably the acetyl residue, or a cyanoalkyl residue, preferably the cyanoethyl residue, or a salt of this compound for the detection of leukocytes and other esterolytically and/or proteolytically effective substances in body liquids and exudations, particularly on an absorbable carrier together with an enzyme-specific substrate and/or chromogen, as well as further auxiliaries, such as buffer systems, optionally thickening agents, surface-active agents, oxidants, and/or stabilizers.

2. Diagnostic, particularly quick diagonistic, in form of a test strip, consisting of a carrier, an enzyme-specific substrate and/or chromogen for the detection of esterolytically or proteolytically effective enzymes, an activator, as well as further auxiliaries, such as a buffer system, optionally thickeners, surface-active agents, oxidants, and/or further stabilizers, characterized in that the activator is a compound according to Claim 1.